# EUROPÄISCHE PATENTSCHRIFT

(11) **EP 1 961 807 B1**
(45) Veröffentlichungstag und Bekanntmachung des Hinweises auf die Patenterteilung: **25.02.2015**
(21) Anmeldenummer: 07003739.5
(22) Anmeldetag: 23.02.2007
(51) Int. Cl.: C12M 3/00, C12M 1/42, G01N 27/447

(54) **Vorrichtung und Verfahren zur Stabilisierung des Flusses durch eine Kammer**
Device and method for stabilising flow through a chamber
Procédé et dispositif destinés à la stabilisation du flux à travers

(43) Veröffentlichungstag der Anmeldung: 27.08.2008
(73) Patentinhaber: Lonza Cologne GmbH, 50829 Köln (DE)
(72) Erfinder: Kazinski, Michael, 50969 Köln (DE); Wirth, Andreas, 9555 Bühler (CH)
(74) Vertreter: Remus, Alvaro Johannes

(56) Entgegenhaltungen:
- US-A- 5 098 843
- US-A- 5 965 410
- US-A- 6 150 148

## Beschreibung

Die Erfindung betrifft eine Vorrichtung, zumindest bestehend aus mindestens einem Eingangskanal zum Einleiten eines Fluids in eine Kammer und mindestens einem Ausgangskanal zum Ableiten des Fluids aus der Kammer, wobei die Kammer mindestens zwei Elektroden zur Erzeugung eines elektrischen Feldes in der Kammer aufweist, die mit dem Innenraum der Kammer in Kontakt stehen. Die Erfindung betrifft ferner ein Verfahren zur Stabilisierung des Flusses eines Fluids durch eine Kammer, die mindestens zwei Elektroden, die mit dem Innenraum der Kammer in Kontakt stehen, sowie mindestens einen Eingangskanal zum Einleiten des Fluids in die Kammer und mindestens einen Ausgangskanal zum Ableiten des Fluids aus der Kammer aufweist, wobei in der Kammer mittels der beiden Elektroden ein elektrisches Feld erzeugt wird.

Das Einbringen biologisch aktiver Moleküle, wie beispielsweise DNA, RNA oder Proteine, in lebende Zellen kann beispielsweise der Untersuchung der biologischen Funktionen dieser Moleküle dienen und ist darüber hinaus unabdingbare Vorraussetzung für den Erfolg eines therapeutischen Einsatzes dieser Moleküle, beispielsweise in der Gentherapie. Eine bevorzugte Methode zum Einbringen von Fremdmolekolen in die Zellen ist dabei die sogenannte Elektroporation, welche im Gegensatz zu chemischen Methoden geringere unerwünschte Veränderungen der biologischen Struktur und Funktionen der Zielzelle bewirkt. Bei der Elektroporation werden die Fremdmoleküle aus einer wässrigen Lösung, vorzugsweise einer an die Zellen angepassten Pufferlösung oder einem Zellkulturmedium, durch einen kurzzeitigen Stromfluss, d.h. beispielsweise den Puls eines sich entladenden Kondensators, in die Zellen eingebracht, wobei durch die Wirkung der kurzen elektrischen Pulse die Zellmembran für die Fremdmoleküle durchlässig gemacht wird. Durch die kurzzeitig entstehenden "Poren" in der Zellmembran gelangen die biologisch aktiven Moleküle zunächst in das Zytoplasma, in dem sie ggf. bereits ihre zu untersuchende Funktion oder therapeutische Wirkung ausüben können, und daraufhin unter bestimmten Bedingungen auch in den Zellkern, was bei gentherapeutischen Anwendungen erforderlich ist. Durch das kurzzeitige Anlegen eines starken elektrischen Feldes, d.h. eines kurzen Pulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldpulses kommt es dann zur Interaktion von Membranteilen, die schließlich zur Fusion führt. Für die Elektrofusion können dabei vergleichbare apparative Vorrichtungen verwendet werden, wie für die Elektroporation.

Kleinere Volumina werden zumeist im Batch-Verfahren in relativ einfachen Gefäßen behandelt. Die Lösung bzw. Zellsuspension befindet sich dabei häufig in einer Küvette, d.h. einem schmalen, nach oben offenen Gefäß, das in der Nähe des Bodens zwei gegenüberliegende, parallele Elektroden in den Seitenwänden aufweist, welche zum Anlegen der elektrischen Spannung dienen. Für die Behandlung größerer Volumina sind solche Gefäße aber ungeeignet, weil der für die elektrische Behandlung zur Verfügung stehende Reaktionsraum nicht beliebig vergrößert werden kann, da der Abstand der Elektroden ein limitierender Faktor ist. Für die Elektroporation bzw. Elektrofusion größerer Volumina werden daher bevorzugt Durchfluss-Verfahren angewendet, bei denen die Zell- oder Vesikelsuspension kontinuierlich oder diskontinuierlich durch den Reaktionsraum zwischen den Elektroden geleitet wird.

Aus der US-A-6 150 148 ist beispielsweise eine für Durchfluss-Verfahren modifizierte Küvette bekannt, deren Öffnung mittels einer Kappe verschlossen ist, durch welche eine Zuleitung geführt ist. Im Bereich des Bodens zwischen den Elektroden weist die Küvette eine zusätzliche Öffnung auf, an die eine Ableitung angeschlossen ist. Durch diese Anordnung kann die zu behandelnde Suspension über die Zuleitung in den Reaktionsraum zwischen den Elektroden geleitet und über die Ableitung wieder ausgeleitet werden. Durch wiederholtes, kontinuierliches oder diskontinuierliches Austauschen der Suspension im Reaktionsraum und entsprechend wiederholtes Pulsen können mit der bekannten Küvette auch größere Volumina behandelt werden.

Die US-A-6 150 148 offenbart ferner Durchfluss-Kammern, die rohr- oder schlitzförmig ausgebildet sind und an ihren Enden jeweils einen Anschluss für einen Eingangs- und einen Ausgangskanal aufweisen. Die Kammern selbst stellten einen länglichen Reaktionsraum dar, der von zwei zylinderförmigen, konzentrisch angeordneten oder flachen, planparallel angeordneten Elektroden eingeschlossen ist. Mittels dieser Vorrichtungen können auch hier größere Volumina durch wiederholtes Pulsen beim Durchleiten durch die Kammer behandelt werden.

Die US-A-5 965 410 offenbart ein Verfahren und eine Vorrichtung zur kontrollierten Erhitzung eines Fluids in einem Mikrofluidsystem. Das System umfasst mindestens zwei über einen Übergangsbereich miteinander verbundene Kanäle, wobei das Fluid in den Kanälen über Elektroden mit Energie bzw. elektrischem Strom beaufschlagt und dabei erhitzt wird. Aufgrund der unterschiedlichen Durchmesser der beiden Kanäle weist das Fluid in den Kanälen unterschiedliche Temperaturen auf.

Bei der Elektroporation oder Elektrofusion im Durchfluss-Verfahren stellt die Entstehung von Gasblasen durch Elektrolyse, neben der Erwärmung der Suspension, ein bedeutendes Problem dar. Aufgrund der häufig für diese Verfahren notwendigen sehr hohen Ströme entstehen in der Elektrolytlösung, in der die zu behandelnden Zellen oder Vesikel suspendiert sind, durch elektrochemische Prozesse viele kleine Gasblasen, die den Fluss der Suspension durch die Kammer stören und zu einem Rückfluss der bereits behandelten Suspension in die Kammer führen können. Dies führt einerseits zu nicht mehr reproduzierbaren Ergebnissen und ferner bei der Behandlung von lebenden Zellen zu einer erhöhten Mortalitätsrate.

Es ist daher Aufgabe der Erfindung, eine Vorrichtung und ein Verfahren für die Durchfluss-Elektroporation oder -Elektrofusion zur Verfügung zu stellen, bei denen ein gerichteter Fluss des zu behandelnden Fluids durch die Kammer bzw. den Reaktionsraum gewährleistet ist und ein Rückfluss des bereits behandelten Fluids in die Kammer bzw. den Reaktionsraum vermieden werden kann.

Die Aufgabe wird erfindungsgemäß dadurch gelöst, dass der durchschnittliche Innendurchmesser des Eingangskanals der eingangs genannten Vorrichtung kleiner als der durchschnittliche Innendurchmesser des Ausgangskanals ist. Die Aufgabe wird hinsichtlich des eingangs genannten Verfahrens erfindungsgemäß ferner dadurch gelöst, dass der Eingangskanal zur Reduzierung seines Innendurchmessers verengt wird. Durch das Vorsehen eines stromaufwärts gelegenen, zugangsseitig geringeren Innendurchmessers erhöht sich der Druck vor der Kammer (stromaufwärts) im Vergleich zum Druck hinter der Kammer (stromabwärts), so dass der Fluss des Fluids insgesamt hinsichtlich seiner Richtung stabilisiert wird. Durch dieses Druckgefälle wird darüber hinaus in vorteilhafter Weise verhindert, dass Gasblasen bzw. Fluid aus dem Ausgangskanal in die Kammer zurückfließen bzw. zurückfließt. Erfindungsgemäß wird der durchschnittliche Innendurchmesser des Eingangskanals im Vergleich zum durchschnittlichen Innendurchmesser des Ausgangskanals so gewählt, dass der Druck stromaufwärts der Kammer immer höher als stromabwärts der Kammer ist. Im einfachsten Fall ist der Innendurchmesser des Eingangskanals über seine gesamte Länge geringer als der Innendurchmesser des Ausgangskanals über dessen gesamte Länge oder an dessen engster Stelle. Der Innendurchmesser des Eingangskanals kann aber beispielsweise, wenn beide Kanäle ansonsten einen ungefähr gleichen Innendurchmesser aufweisen, gegenüber dem Innendurchmesser des Ausgangskanals auch an einer Stelle oder innerhalb eines begrenzten Abschnitts verengt sein bzw. werden. Die Verengung des Innendurchmessers des Eingangskanals stellt dabei eine einfache und kostengünstige Maßnahme dar, die die Verwendung komplizierter, elektronisch gesteuerter Ventilsysteme überflüssig macht.

Die unabhängigen Ansprüche 1 und 12 lauten wie folgt:
1. Vorrichtung (1, 50, 60), zumindest bestehend aus mindestens einem Eingangskanal (6, 51, 65) zum Einleiten eines Fluids in eine Kammer (7, 54, 62) und mindestens einem Ausgangskanal (11, 52, 66) zum Ableiten des Fluids aus der Kammer (7, 54, 62), wobei die Kammer (7, 54, 62) mindestens zwei Elektroden (8) zur Erzeugung eines elektrischen Feldes in der Kammer (7, 54, 62) aufweist, die mit dem Innenraum (10, 53, 67) der Kammer (7, 54, 62) in Kontakt stehen, dadurch gekennzeichnet, dass der durchschnittliche Innendurchmesser des Eingangskanals (6, 51, 65) kleiner als der durchschnittliche Innendurchmesser des Ausgangskanals (11,52, 66) ist.
12. Verfahren zur Stabilisierung des Flusses eines Fluids durch eine Kammer (7, 54, 62), die mindestens zwei Elektroden (8), die mit dem Innenraum (10, 53, 67) der Kammer (7, 54, 62) in Kontakt stehen, sowie mindestens einen Eingangskanal (6, 51, 65) zum Einleiten des Fluids in die Kammer (7, 54, 62) und mindestens einen Ausgangskanal (11, 52, 66) zum Ableiten des Fluids aus der Kammer (7, 54, 62) aufweist, wobei in der Kammer (7, 54, 62) mittels der beiden Elektroden (8) ein elektrisches Feld erzeugt wird, dadurch gekennzeichnet, dass der Eingangskanal (6, 51, 65) Reduzierung seines Innendurchmessers verengt wird und dadurch der durchschnittliche Innendurchmesser des Eingangskanals (6, 51, 65) kleiner als der durchschnittliche Innendurchmesser des Ausgangskanals (11,52, 66) ist.

Bevorzugte Ausführungen der beanspruchten Erfindung finden sich in den abhängigen Ansprüchen 2-11 und 13-16.

In vorteilhafter Ausgestaltung der erfindungsgemäßen Vorrichtung ist der Innendurchmesser des Eingangskanals an mindestens einer Stelle geringer als der kleinste Innendurchmesser des Ausgangskanals. Der Innendurchmesser des Eingangskanals kann also über die gesamte Länge desselben oder nur innerhalb mindestens eines Abschnitts oder an mindestens einer Stelle geringer als die engste Stelle des Ausgangskanals sein. Bei dieser Ausführungsform kann auf relativ einfache Weise sichergestellt werden, dass der Druck in Flussrichtung vor der Kammer in jedem Fall höher als hinter der Kammer ist.

Der Eingangskanal kann beispielsweise mindestens eine Verengung zur Reduzierung seines Innendurchmessers aufweisen. Dabei kann die Verengung durch Reduzierung des Außendurchmessers des Eingangskanals bei zumindest annähernd gleichbleibender Wandstärke und/oder durch innerhalb des Eingangskanals angeordnete Erhebungen, Wulste oder Ähnliches gebildet sein. Auch das Einfügen von Schläuchen oder Röhrchen mit geringerem Durchmesser in den Eingangskanal ist möglich. Ferner kann in besonders vorteilhafter Ausgestaltung der Erfindung auch eine variable Verschlusseinrichtung, wie beispielsweise ein Schieber, eine Klappe, ein Ventil oder eine Blende, in oder an den Eingangskanal angebracht sein, mit deren Hilfe der Innendurchmesser des Eingangskanals manuell oder automatisch eingestellt werden kann.

In weiterer vorteilhafter Ausgestaltung der erfindungsgemäßen Vorrichtung ist vorgesehen, dass der Ausgangskanal bogen- oder kurvenförmig ausgebildet ist, um das Entstehen von Kanten und/oder Todräumen, an bzw. in denen sich Zellbestandteile oder -trümmer festsetzen könnten, zu vermeiden. Da die Behandlung insbesondere von lebenden Zellen mit elektrischem Strom bzw. das Durchleiten derselben durch ein elektrisches Feld immer zu einer Antötung bzw. Zerstörung einzelner Zellen führt, befinden sich im stromabwärts von der Kammer gelegenen Ausgangskanal im Fluid neben intakten Zellen auch Zelltrümmer und demzufolge auch intrazelluläre Bestandteile, die sich an geeigneten Stellen ablagern und somit zu einer Verstopfung des Ausgangskanals führen könnten. Um dies zu vermeiden sollte der Ausgangskanal keine rechtwinkligen Richtungsänderungen aufweisen, auch wenn eine Richtungsänderung aus konstruktiven Gründen erforderlich sein sollte.

Damit die im Fluid durch die elektrische Behandlung entstehenden Gasblasen problemlos entweichen können, sollte die Mündung des Ausgangskanals vorzugsweise oberhalb der Kammer angeordnet sein. Folglich sollte auch der Ausgangskanal vorzugsweise oberhalb des Eingangskanals und nicht auf gleicher Höhe mit diesem angeordnet sein.

Die erfindungsgemäße Vorrichtung kann ferner ein Gehäuse aufweisen, wobei der Ausgangs- und der Eingangskanal aus Sicherheitsgründen an der Oberfläche oder außerhalb des Gehäuses münden. Durch diese Konstruktion ist sichergestellt, dass eine die Vorrichtung bedienende Person nicht in den Innenraum des Gehäuses, in dem sich die Elektroden und andere möglicherweise elektrisch geladene Bauteile befinden, eingreifen kann oder muss. Der Anschluss eines Reservoirs und eines Auffangbehälters ist also bei einer solchen Vorrichtung gefahrlos möglich. Dabei sollten die Mündungen beider Kanäle aus praktischen Gründen auf der gleichen Seite des Gehäuses liegen, vorzugsweise der Vorder- bzw. Frontseite, um die Handhabung der erfindungsgemäßen Vorrichtung zu vereinfachen.

Zusätzlich zu den beschriebenen Maßnahmen kann/können der Ausgangskanal und/oder der Eingangskanal mit mindestens einem aktiv gesteuerten oder passiven Ventil, vorzugsweise mindestens einem Rückschlagventil, versehen sein. Hierdurch kann für den Fall, dass aus irgendwelchen Gründen kurzzeitig eine Umkehr der Druckverhältnisse auftritt, ein Rückfluss des Fluids in die Kammer verhindert werden.

Aus Sicherheitsgründen ist erfindungsgemäß ferner vorgesehen, dass der Eingangskanal und/oder der Ausgangskanal mit mindestens einer Erdungselektrode versehen ist/sind. Auf diese Weise kann verhindert werden, dass die hohen Ströme, die bei vielen Anwendungen in der erfindungsgemäßen Vorrichtung fließen, eine Gefährdung des Bedienpersonals darstellen. Eine oder mehrere Erdungselektroden können dabei an einer beliebigen Stelle stromabwärts, stromaufwärts und/oder direkt an der Kammer angeordnet sein.

In besonders vorteilhafter Ausgestaltung der erfindungsgemäßen Vorrichtung ist vorgesehen, dass die mindestens zwei Elektroden an ihrer vom Fluid abgewandten Seite mit mindestens einer Kühleinrichtung versehen sind. Da beim Durchfluss größerer Mengen an Fluid durch die Kammer eine Vielzahl von Spannungspulsen erzeugt werden muss, kommt es zwangsläufig zu einer Erwärmung der Elektroden und damit auch des Fluids. Um diese Erwärmung in Grenzen zu halten und einen Anstieg der Fluidtemperatur auf eine für das zu behandelnde biologische Material schädliche Temperatur zu verhindern, ist eine Kühlung zumindest der Elektroden erforderlich. Beispielsweise bei der Behandlung von lebenden Zellen mit elektrischem Strom sollte die Temperatur nicht über 40°C ansteigen. Die Kühlung der Elektroden kann beispielsweise durch einen herkömmlichen CPU-Kühler, ein Peltier-Element oder Ähnliches erfolgen. Aus Sicherheitsgründen kann zur Isolierung dabei in vorteilhafter Ausgestaltung der Erfindung zwischen der jeweiligen Elektrode und der Kühleinrichtung eine nicht-stromleitende, wärmeleitende Folie angeordnet sein.

Die Erfindung betrifft auch die vorteilhafte Verwendung der erfindungsgemäßen Vorrichtung für die Elektroporation oder Elektrofusion von Zellen, Zellpartikeln und/oder Membranvesikeln im Durchflussverfahren. Die erfindungsgemäße Vorrichtung erlaubt dabei in vorteilhafter Weise die Behandlung großer Volumina des biologischen Materials durch kontinuierliches oder diskontinuierliches Durchleiten der jeweiligen Suspension durch die Kammer.

In vorteilhafter Ausgestaltung des erfindungsgemäßen Verfahrens ist vorgesehen, dass die Verengung durch Reduzierung des Außendurchmessers des Eingangskanals bei zumindest annähernd gleich bleibender Wandstärke und/oder durch Einfügen von Erhebungen, Wulsten oder Ähnlichem in den Eingangskanal hergestellt wird.

Besonders bevorzugt ist eine Ausführungsform des erfindungsgemäßen Verfahrens, bei der die Reduzierung des Innendurchmessers des Eingangskanals variiert wird. Durch manuelle oder automatische Betätigung eines Schiebers, einer Klappe, eines Ventils, einer Blende oder einer ähnlichen Verschlusseinrichtung, kann der Innendurchmesser des Eingangskanals dabei so eingestellt werden, dass die Druckverhältnisse in der erfindungsgemäßen Vorrichtung optimal an die jeweiligen Bedingungen angepasst sind. Auf diese Weise kann der Fluss des Fluids durch Variieren des Innendurchmessers des Eingangskanals gesteuert werden, um das Einstellen eines optimalen Verhältnisses zwischen stromaufwärtigem Überdruck einerseits und der Fliessgeschwindigkeit andererseits unter unterschiedlichen Bedingungen zu ermöglichen.

Um eine Erwärmung des Fuids beim Durchfluss durch das elektrische Feld in der Kammer in Grenzen zu halten und einen Anstieg der Fluidtemperatur auf eine für das zu behandelnde Material schädliche Temperatur zu verhindern, ist vorgesehen, dass mindestens eine Elektrode, die zur Erzeugung des elektrischen Feldes in der Kammer vorgesehen ist, gekühlt wird.

Das Fluid kann bei dem erfindungsgemäßen Verfahren kontinuierlich oder diskontinuierlich durch die Kammer geleitet werden. Dabei wird eine Serie von elektrischen Feldern in der Kammer erzeugt, damit jedes Teilvolumen des Fluids dem elektrischen Feld ausgesetzt ist. Die Fliessgeschwindigkeit muss auf die Abstände zwischen den einzelnen Spannungspulsen, oder umgekehrt die Abstände zwischen den einzelnen Spannungspulsen auf die Fliessgeschwindigkeit, derart abgestimmt werden, dass ein Teilvolumen des zu behandelnden Fluids möglichst genau der gewünschten Anzahl an elektrischen Pulsen ausgesetzt ist. Jedenfalls sollte vermieden werden, dass ein zu großer Anteil des zu behandelnden Fluids den Spannungspulsen häufiger als erwünscht oder gar nicht ausgesetzt ist. Vorzugsweise enthält das Fluid Zellen, Zellpartikel und/oder Membranvesikel, welche beim Durchleiten durch die Kammer mindestens einem elektrischen Feld ausgesetzt werden.

Die Erfindung wird im Weiteren anhand der folgenden Abbildungen beispielhaft näher erläutert. Es zeigt
- Figur 1: einen schematischen Längsschnitt durch eine Ausführungsform der erfindungsgemäßen Vorrichtung,
- Figur 2: die Vorrichtung gemäß Figur 1 mit zusätzlicher Verengung des Eingangskanals,
- Figur 3: die Vorrichtung gemäß Figur 1 mit Erdungselektroden,
- Figur 4: die Vorrichtung gemäß Figur 2 mit Erdungselektroden,
- Figur 5: einen schematischen Längsschnitt durch weitere Ausführungsformen der erfindungsgemäßen Vorrichtung mit unterschiedlichen Verengungen des Eingangskanals und
- Figur 6: eine weitere Ausführungsform der erfindungemäßen Vorrichtung mit Erdungselektroden.

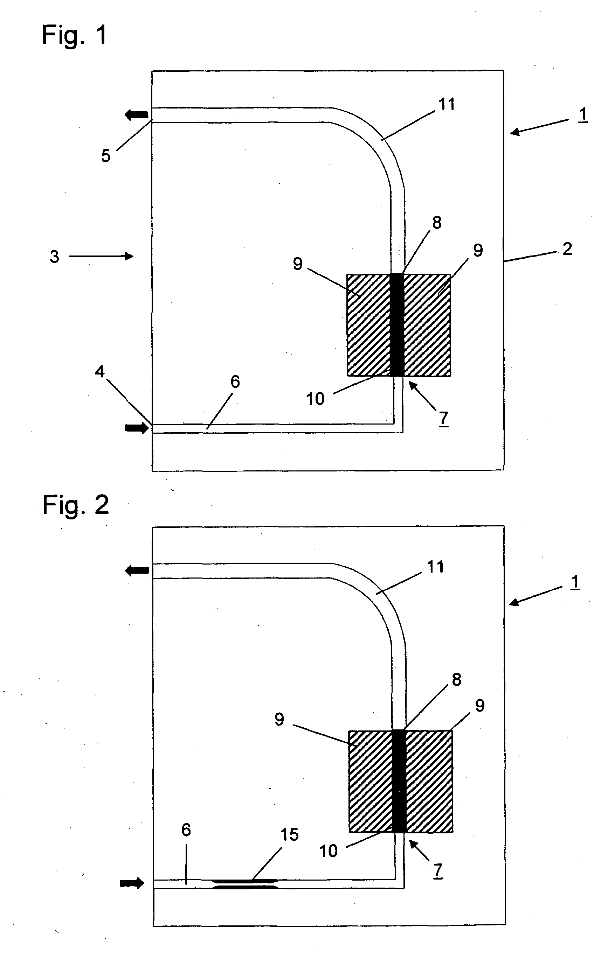

Figur 1 zeigt einen Längsschnitt durch eine beispielhafte Ausführungsform der erfindungsgemäßen Vorrichtung. Die Vorrichtung 1 umfasst ein Gehäuse 2, das an seiner Vorderseite 3 zwei Öffnungen 4, 5 aufweist. An die Öffnungen 4, 5 können, vorzugsweise über Schläuche oder sonstige Leitungen, Gefäße angeschlossen werden, die jeweils der Aufnahme eines Fluids dienen. So kann beispielsweise an die Öffnung 4 ein Vorratsgefäß oder Reservoir angeschlossen werden, aus dem das Fluid, vorzugsweise mittels einer Pumpe oder Druckluft, in den Eingangskanal 6 der Vorrichtung 1 eingebracht wird (Die Pfeile an den Öffnungen 4, 5 zeigen die Flussrichtung des Fluids an). Über den Eingangskanal 6 gelangt das Fluid dann in eine Kammer 7, die zwei Elektroden aufweist, welche mit dem Innenraum 10 der Kammer 7 in Kontakt stehen. Von den Elektroden ist in dieser Darstellung nur die hinter dem Innenraum 10 der Kammer 7 angeordnete Elektrode 8 sichtbar, während die zweite Elektrode, die zu der Elektrode 8 planparallel angeordnet ist, vor der Schnittebene liegt. Die beiden Elektroden sind durch zwei Isolatoren 9 voneinander getrennt, wobei die beiden Isolatoren in der Verlängerung des Eingangskanals 6 einen Spalt bilden, der den Innenraum 10 der Kammer 7 darstellt. Der Innenraum 10 der Kammer 7 wird also von der Vorderseite 3 der Vorrichtung 1 aus betrachtet seitlich durch die Elektroden sowie vorne und hinten durch die zwischen den Elektroden liegenden Isolatoren 9 begrenzt. Beim Anlegen einer elektrischen Spannung an die Elektroden entsteht im Innenraum 10 ein elektrisches Feld, das auf das im Fluid befindliche Material einwirkt. Die angelegte Spannung ist vorzugsweise ein Hochspannungspuls, der durch die Entladung eines oder mehrerer Kondensatoren generiert wird. Durch die Entladung des Kondensators entsteht zwischen den Elektroden ein kurzzeitiges, starkes elektrisches Feld im Innenraum 10 der Kammer 7.

Wenn das Fluid beispielsweise eine Suspension ist, bei der Zellen, Zellpartikel und/oder Membranvesikel in einer Elektrolytlösung suspendiert sowie biologisch aktive Moleküle gelöst sind, so können diese biologisch aktiven Moleküle mit Hilfe des elektrischen Feldes in die Zellen, Zellpartikel und/oder Membranvesikel eingebracht werden (Elektroporation). Alternativ können die Zellen, Zellpartikel und/oder Membranvesikel durch das elektrische Feld auch fusioniert werden (Elektrofusion). Werden größere Volumina des Fluids bzw. der Suspension kontinuierlich oder diskontinuierlich durch die Kammer 7 geleitet, so müssen elektrische Felder in kurzen Abständen erzeugt werden, so dass jedes Teilvolumen des Fluids bzw. der Suspension mindestens einem elektrischen Feld ausgesetzt ist und wie gewünscht behandelt werden kann.

Aus der Kammer 7 gelangt das behandelte Material dann in den Ausgangskanal 11 der Vorrichtung 1. Der Ausgangskanal 11 ist bogenförmig ausgebildet und mündet daher in die Öffnung 5 an der Vorderseite 3 der Vorrichtung 1. Da das Fluid beispielsweise im Falle einer Zellsuspension nach der elektrischen Behandlung in der Kammer 7 auch Zelltrümmer enthält, könnte eine rechtwinklige Ausgestaltung des Ausgangskanals an den dann vorliegenden Ecken und Kanten zu Ablagerungen und im schlimmsten Fall zur Verstopfung des Ausgangskanals führen. Durch die abgerundete Ausgestaltung des Ausgangskanals 11 der Vorrichtung 1 können solche Probleme effektiv vermieden werden, da sich in den Kurvenbereichen des Ausgangskanals 11 keine Zelltrümmer absetzen können. An die Öffnung 5 kann beispielsweise ein Auffang- oder Sammelbehälter angeschlossen werden, in dem das Fluid bzw. die Suspension nach der Behandlung in der Vorrichtung 1 aufgefangen werden kann. Mit an die Öffnungen 4, 5 angeschlossenen Vorrats- und Auffanggefäßen stellt die erfindungsgemäße Vorrichtung 1 also ein geschlossenes System dar, das auch steril betrieben werden kann.

Durch das elektrische Feld im Innenraum 10 der Kammer 7 zwischen den Elektroden können im Fluid durch Elektrolyse Gasblasen entstehen, die den Fluss des Fluids stören können. Insbesondere kann es bei einer Vielzahl von Hochspannungspulsen zu einem Überdruck in der Kammer 7 kommen, der die Gasblasen und bereits behandeltes Material zurück in den Eingangskanal 6 drückt. Um diesen unerwünschten Effekt zu vermeiden, ist bei der erfindungsgemäßen Vorrichtung 1 der durchschnittliche Innendurchmesser des Eingangskanals 6 kleiner als der durchschnittliche Innendurchmesser des Ausgangskanals 11. In der hier dargestellten Ausführungsform haben sowohl der Eingangskanal 6 als auch der Ausgangskanal 11 einen über ihre gesamte Länge jeweils in etwa konstanten Innendurchmesser. Dadurch, dass der Innendurchmesser des Eingangskanals 6 deutlich kleiner als der Innendurchmesser des Ausgangskanals 11 ist, entsteht beim Durchfluss des Fluids stromaufwärts der Kammer 7 im Eingangskanal 6 ein höherer Druck als stromabwärts der Kammer 7 im Ausgangskanal 11. Dieses Druckgefälle führt in vorteilhafter Weise dazu, dass der Fluss des Fluids konstant in Richtung der Pfeile gerichtet bleibt und kein bereits behandeltes Fluid in die Kammer 7 oder den Eingangskanal 6 zurückfließen kann.

Als zusätzliche vorteilhafte Maßnahme liegt bei der erfindungsgemäßen Vorrichtung 1 die Mündung bzw. Öffnung 5 des Ausgangskanals 11 oberhalb des Eingangskanals 6 und auch oberhalb der Kammer 7. Hierdurch wird das Entweichen der Gasblasen aus dem Ausgangskanal 11 erleichtert. Darüber hinaus kann im Ausgangskanal 11, vorzugsweise in der Nähe der Kammer 7, ein Rückschlagventil (hier nicht dargestellt) angeordnet sein, das bei einer kurzeitigen Druckumkehr, beispielsweise beim Abschalten der Pumpe oder zwischen den Spannungspulsen, einen Rückfluss des Fluids verhindert. Da die Öffnungen 4, 5 der Kanäle 6, 11 beide an der Vorderseite 3 der Vorrichtung 1 angeordnet sind, können die erforderliche Gefäße auf einfache Weise an die Vorrichtung 1 angeschlossen werden. Die Vorrichtung 1 ist also sehr bedienerfreundlich und darüber hinaus auch sehr sicher, da die erforderlichen Gefäße bzw. Schläuche nicht direkt an die Kammer 7, sondern an das Gehäuse 2 angeschlossen werden können. Eine Gefährdung des Bedienpersonals durch die Spannungsquelle oder die Elektroden kann daher ausgeschlossen werden.

Figur 2 zeigt die Vorrichtung 1 gemäß Figur 1 mit zusätzlich in den Eingangskanal 6 eingefügter Verengung 15. Somit ist bei dieser Ausführungsform der Innendurchmesser des Eingangskanals 6 im Bereich der Verengung 15 nochmals gegenüber dem Innendurchmesser des Ausgangskanals 11 verringert, so dass das Druckgefälle weiter vergrößert ist. Durch die zusätzliche Erhöhung des Fluiddrucks im Eingangskanal 6 kann selbst bei sehr starker Gasbildung in der Kammer 7 ein Rückfluss von Fluid in den Innenraum 10 der Kammer 7 bzw. den Eingangskanal 6 verhindert werden. Bei der Verengung 15 handelt es sich um eine nach innen gerichtete, zylinderförmige Verdickung der Wand des Eingangskanals 6 bei gleich bleibendem Außendurchmesser. Die Verengung 15 ist also eine Art Wulst oder Erhebung der Innenwand des Eingangskanals 6. Alternativ kann der gleiche Effekt beispielsweise auch durch das Einbringen eines kurzen Schlauchstücks in den Eingangskanal 6 erzielt werden, wobei der Außendurchmesser des Schlauchsstücks gleich oder etwas größer als der Innendurchmesser des Eingangskanals sein sollte (Presspassung).

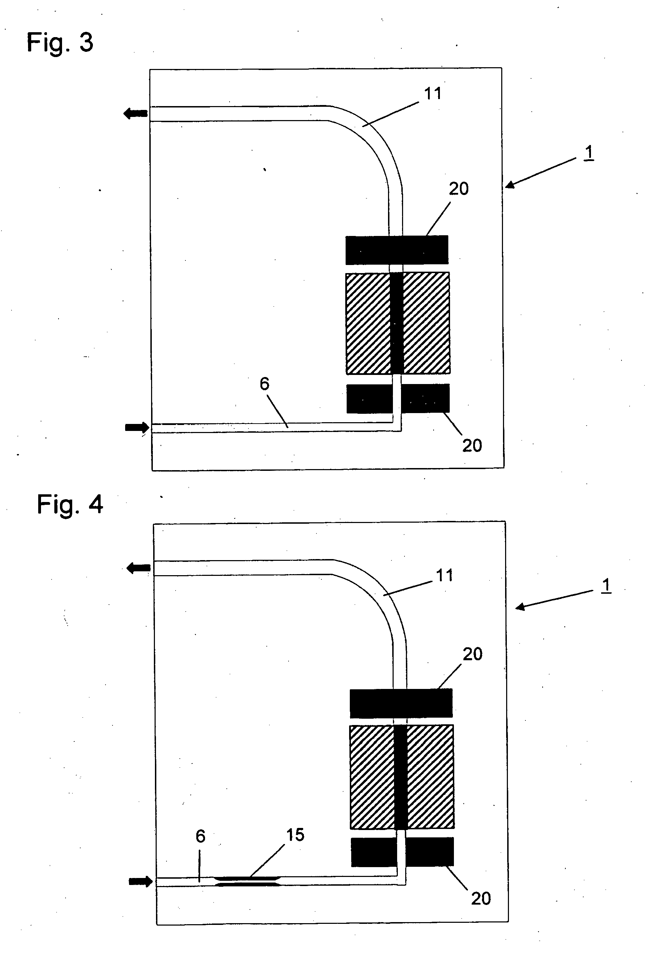

Die Figuren 3 und 4 zeigen die Vorrichtungen 1 gemäß der Figuren 1 und 2 jeweils mit Erdungselektroden 20. Die Erdungselektroden dienen der Sicherheit der erfindungsgemäßen Vorrichtung 1. Die hohen Ströme, die bei vielen Anwendungen in der erfindungsgemäßen Vorrichtung 1 fließen, stellen eine permanente Gefahr für das Bedienpersonal dar. Eine oder mehrere Erdungselektroden können an einer beliebigen Stelle stromabwärts, stromaufwärts und/oder direkt an der Kammer angeordnet sein, um die Gefahr eines Stromschlags zu reduzieren. Die Erdungselektroden 20 sind beidseits der Kammer 7 jeweils am Eingangs- und Ausgangskanal 6, 11 angebracht, so dass aus der Kammer 7 fließende Ströme direkt abgeleitet werden können.

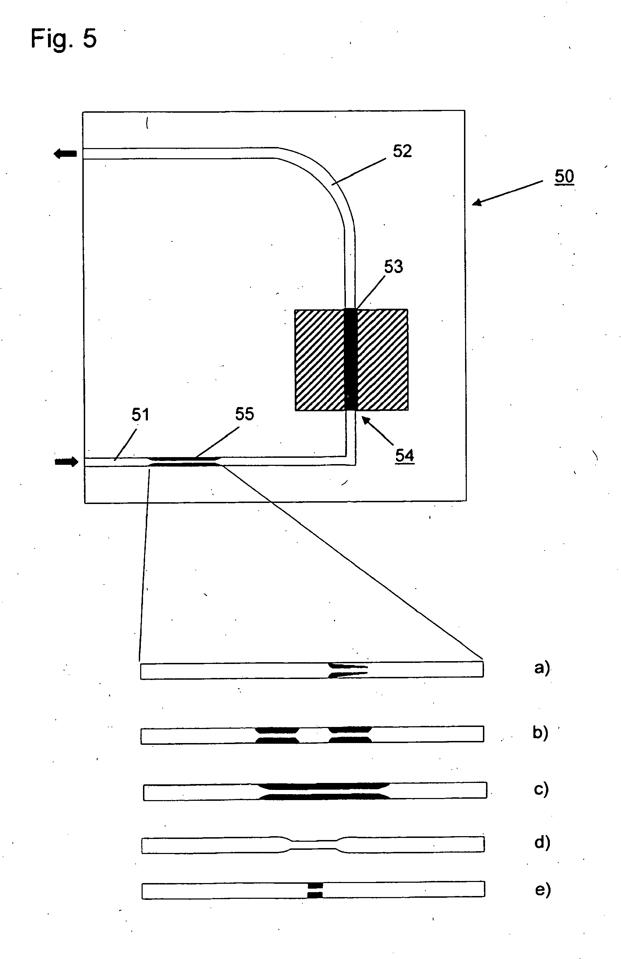

Figur 5 zeigt einen Längsschnitt durch eine weitere Ausführungsform der erfindungsgemäßen Vorrichtung mit unterschiedlichen Verengungen des Eingangskanals. Die Vorrichtung 50 weist im Unterschied zur Vorrichtung 1 gemäß der Figuren 1 bis 4 einen Eingangskanal 51 und einen Ausgangskanal 52 mit annähernd gleichem Innen- (und Außen-) Durchmesser auf. Der Druck, der durch die Spannungspulse und die hierdurch entstehenden Gasblasen im Innenraum 53 der Kammer 54 und im Ausgangskanal 52 entsteht, wird durch eine Verengung 55 im Eingangskanal 51 überkompensiert, die im wesentlichen der Verengung 15 gemäß der Figuren 2 und 4 entspricht. Aufgrund der durch die Verengung 55 hervorgerufenen Verringerung des durchschnittlichen Innendurchmessers des Eingangskanals 51 im Vergleich zum durchschnittlichen Innendurchmesser des Ausgangskanals 52, kann ein gerichteter Fluss des Fluids durch die Kammer 54 auch bei starker Gasbildung aufrechterhalten werden. Die Verringerung des durchschnittlichen Innendurchmessers des Eingangskanals 51 kann dabei auf unterschiedliche Weise erreicht werden, wie die verschiedenen Ausführungsformen gemäß a) bis e) zeigen. Während die Ausführungsformen gemäß b) und e) im Prinzip der Ausführungsform gemäß c) ähnlich sind, wird bei der Ausführungsform gemäß d) die Verengung durch eine Reduzierung des Außendurchmessers des Eingangskanals bei gleich bleibender Wandstärke erreicht. Eine solche Wirkung kann beispielsweise auch durch das Anbringen einer Klemme oder Ähnlichem an einen nachgiebigen Abschnitt des Eingangskanals erzielt werden. In diesem Fall könnte die Reduzierung des Innendurchmessers und damit auch der Druck im Eingangskanal flexibel variiert werden. Die Ausführungsform gemäß a) zeigt ein Ventil, wobei es sich hierbei um ein aktiv gesteuertes oder passives Ventil handeln kann. Im Fall eines gesteuerten Ventils ist es ebenfalls möglich, die Reduzierung des Innendurchmessers und damit auch den Druck im Eingangskanal flexibel zu variieren. Das Ventil könnte in vorteilhafter Weise auch mit den Spannungspulsen synchronisiert werden, um das erfindungsgemäße Verfahren weiter zu optimieren.

Vorzugsweise sind die Übergänge der Innenwand des Eingangskanals zum Bereich der Verengung abgerundet bzw. abgeflacht ausgebildet, wie beispielsweise bei den Ausführungsformen gemäß Figur 5 a) bis d), um scharfkantige Übergänge zu vermeiden. Dies ist besonders vorteilhaft, da ansonsten das zu behandelnde Material durch zu starke Scherkräfte geschädigt werden könnte.

Die Verengungen 55 können selbstverständlich an unterschiedlichen Stellen des Eingangskanals angeordnet sein. Darüber hinaus ist es auch möglich mehrere Verengungen in Reihe anzuordnen, um deren Wirkung zu erhöhen.

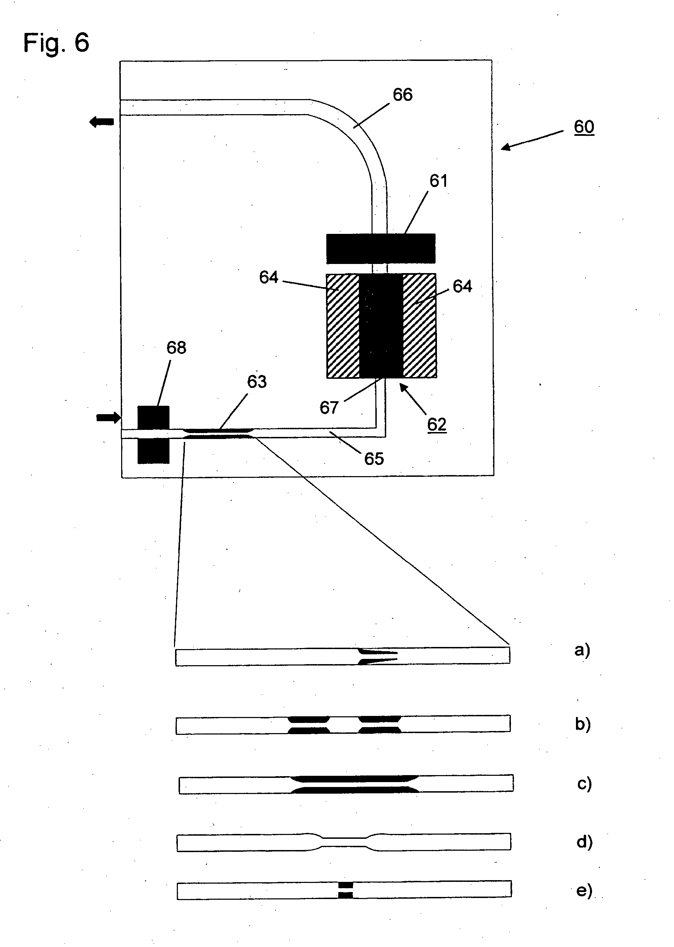

Figur 6 zeigt eine weitere erfindungsgemäße Vorrichtung 60, die im Prinzip der Vorrichtung 1 gemäß Figur 1 mit zusätzlich angebrachten Erdungselektroden 60, 61 entspricht. Bei dieser vorteilhaften Ausführungsform wird deutlich, dass die Erdungselektroden an unterschiedlichen Stellen entlang des Eingangs- und Ausgangskanals angeordnet sein können. So ist im vorliegenden Beispiel die Erdungselektrode 61 stromaufwärts unmittelbar an der Kammer 62 angeordnet, während die Erdungselektrode 68 stromaufwärts in der Nähe der Verengung 63 angeordnet ist. Darüber hinaus in bei dieser Ausführungsform der Spalt zwischen den Isolatoren 64 breiter als die Innendurchmesser des Eingangskanals 65 und des Ausgangskanals 66, so dass sich die Fliessgeschwindigkeit des Fluids in der Kammer 62 verringert, was zu einer Verlängerung der Verweildauer des Fluids im Innenraum 67 der Kammer 62 führt.

## Patentansprüche

1. Vorrichtung (1, 50, 60), zumindest bestehend aus mindestens einem Eingangskanal (6, 51, 65) zum Einleiten eines Fluids in eine Kammer (7, 54, 62) und mindestens einem Ausgangskanal (11, 52, 66) zum Ableiten des Fluids aus der Kammer (7, 54, 62), wobei die Kammer (7, 54, 62) mindestens zwei Elektroden (8) zur Erzeugung eines elektrischen Feldes in der Kammer (7, 54, 62) aufweist, die mit dem Innenraum (10, 53, 67) der Kammer (7, 54, 62) in Kontakt stehen, **dadurch gekennzeichnet, dass** der durchschnittliche Innendurchmesser des Eingangskanals (6, 51, 65) kleiner als der durchschnittliche Innendurchmesser des Ausgangskanals (11, 52, 66) ist.

2. Vorrichtung nach Anspruch 1, **dadurch gekennzeichnet, dass** der Innendurchmesser des Eingangskanals (6, 51, 65) an mindestens einer Stelle geringer als der kleinste Innendurchmesser des Ausgangskanals (11, 52, 66) ist.

3. Vorrichtung nach Anspruch 1 oder 2, **dadurch gekennzeichnet, dass** der Eingangskanal (6, 51, 65) mindestens eine Verengung (15, 55, 63) zur Reduzierung seines Innendurchmessers aufweist.

4. Vorrichtung nach Anspruch 3, **dadurch gekennzeichnet, dass** die Verengung (15, 55, 63) durch Reduzierung des Außendurchmessers des Eingangskanals (6, 51, 65) bei zumindest annähernd gleichbleibender Wandstärke und/oder durch innerhalb des Eingangskanals (6, 51, 65) angeordnete Erhebungen, Wulste oder Ähnliches gebildet ist.

5. Vorrichtung nach einem oder mehreren der Ansprüche 1 bis 4, **dadurch gekennzeichnet, dass** der Ausgangskanal (11, 52, 66) bogen- oder kurvenförmig ausgebildet ist.

6. Vorrichtung nach einem oder mehreren der Ansprüche 1 bis 5, **dadurch gekennzeichnet, dass** die Mündung des Ausgangskanals (11, 52, 66) oberhalb der Kammer (7, 54, 62) angeordnet ist.

7. Vorrichtung nach einem oder mehreren der Ansprüche 1 bis 6, **dadurch gekennzeichnet, dass** der Ausgangskanal (11, 52, 66) und/oder der Eingangskanal (6, 51, 65) mit mindestens einem Ventil, vorzugsweise mindestens einem Rückschlagventil, versehen ist/sind.

8. Vorrichtung nach einem oder mehreren der Ansprüche 1 bis 7, **dadurch gekennzeichnet, dass** der Eingangskanal (6, 51, 65) und/oder der Ausgangskanal (11, 52, 66) mit mindestens einer Erdungselektrode (20, 61, 68) versehen ist/sind.

9. Vorrichtung nach einem oder mehreren der Ansprüche 1 bis 8, **dadurch gekennzeichnet, dass** die mindestens zwei Elektroden (8) an ihrer vom Fluid abgewandten Seite mit mindestens einer Kühleinrichtung versehen sind.

10. Vorrichtung nach Anspruch 9, **dadurch gekennzeichnet, dass** zwischen der Elektrode (8) und der Kühleinrichtung eine nicht-stromleitende, wärmeleitende Folie angeordnet ist.

11. Verwendung der Vorrichtung nach einem oder mehreren der Ansprüche 1 bis 10 zur Elektroporation oder Elektrofusion von Zellen, Zellpartikeln und/oder Membranvesikeln im Durchflussverfahren.

12. Verfahren zur Stabilisierung des Flusses eines Fluids durch eine Kammer (7, 54, 62), die mindestens zwei Elektroden (8), die mit dem Innenraum (10, 53, 67) der Kammer (7, 54, 62) in Kontakt stehen, sowie mindestens einen Eingangskanal (6, 51, 65) zum Einleiten des Fluids in die Kammer (7, 54, 62) und mindestens einen Ausgangskanal (11, 52, 66) zum Ableiten des Fluids aus der Kammer (7, 54, 62) aufweist, wobei in der Kammer (7, 54, 62) mittels der beiden Elektroden (8) ein elektrisches Feld erzeugt wird, **dadurch gekennzeichnet, dass** der Eingangskanal (6, 51, 65) zur Reduzierung seines Innendurchmessers verengt wird und dadurch der durchschnittliche Innendurchmesser des Eingangskanals (6, 51, 65) kleiner als der durchschnittliche Innendurchmesser des Ausgangskanals (11, 52, 66) ist.

13. Verfahren nach Anspruch 12, **dadurch gekennzeichnet, dass** die Verengung durch Reduzierung des Außendurchmessers des Eingangskanals (6, 51, 65) bei zumindest annähernd gleich bleibender Wandstärke und/oder durch Einfügen von Erhebungen, Wulsten oder Ähnlichem in den Eingangskanal (6, 51, 65) hergestellt wird.

14. Verfahren nach einem oder mehreren der Ansprüche 12 oder 13, **dadurch gekennzeichnet, dass** die Reduzierung des Innendurchmessers des Eingangskanals (6, 51, 85) variiert wird.

15. Verfahren nach einem oder mehreren der Ansprüche 12 bis 14, **dadurch gekennzeichnet, dass** der Fluss des Fluids durch Variieren des Innendurchmessers des Eingangskanals (6, 51, 65) gesteuert wird.

16. Verfahren nach einem oder mehreren der Ansprüche 12 bis 15, **dadurch gekennzeichnet, dass** mindestens eine Elektrode (8), die zur Erzeugung des elektrischen Feldes in der Kammer (7, 54, 62) vorgesehen ist, gekühlt wird.

## Claims

1. Device (1, 50, 60), at least consisting of at least one input channel (6, 51, 65) for introducing a fluid into a chamber (7, 54, 62) and at least one output channel (11, 52, 66) for discharging the fluid from the chamber (7, 54, 62), wherein the chamber (7, 54, 62) has at least two electrodes (8) for generating an electrical field in the chamber (7, 54, 62), which are in contact with the interior (10, 53, 67) of the chamber (7, 54, 62), **characterised in that** the average inside diameter of the input channel (6, 51, 65) is smaller than the average inside diameter of the output channel (11, 52, 66).

2. The device according to Claim 1, **characterised in that** the inside diameter of the input channel (6, 51, 65) is at least at one point smaller than the smallest inside diameter of the output channel (11, 52, 66).

3. The device according to Claim 1 or 2, **characterised in that** the input channel (6, 51, 65) has at least one diminution (15, 55, 63) for reducing its inside diameter.

4. The device according to Claim 3, **characterised in that** the diminution (15, 55, 63) is established by reducing the outside diameter of the input channel (6, 51, 65) while at least approximately maintaining the wall thickness, and/or by elevations, bulges or the like arranged inside the input channel (6, 51, 65).

5. The device according to one or more of Claims 1 to 4, **characterised in that** the output channel (11, 52, 66) is designed in the shape of an arc or curve.

6. The device according to one or more of Claims 1 to 5, **characterised in that** the opening of the output channel (11, 52, 66) is arranged above the chamber (7, 54, 62).

7. The device according to one or more of Claims 1 to 6, **characterised in that** the output channel (11, 52, 66) and/or the input channel (6, 51, 65) is/are provided with at least one valve, preferably at least one check valve.

8. The device according to one or more of Claims 1 to 7, **characterised in that** the input channel (6, 51, 65) and/or the output channel (11, 52, 66) is/are provided with at least one ground electrode (20, 61, 68).

9. The device according to one or more of Claims 1 to 8, **characterised in that** the at least two electrodes (8) are provided with at least one cooling device on their side facing away from the fluid.

10. The device according to Claim 9, **characterised in that** a non-current conducting, heat conducting foil is arranged between the electrode (8) and the cooling device.

11. Use of the device according to one or more of Claims 1 to 10 for the electroporation or electrofusion of cells, cell particles and/or membrane vesicles in a flow-through process.

12. A method for stabilising the flow of a fluid through a chamber (7, 54, 62) which has at least two electrodes (8) being in contact with the interior (10, 53, 67) of the chamber (7, 54, 62), and at least one input channel (6, 51, 65) for introducing the fluid into the chamber (7, 54, 62) as well as at least one output channel (11, 52, 66) for discharging the fluid from the chamber (7, 54, 62), wherein an electrical field is generated in the chamber (7, 54, 62) by means of said two electrodes (8), **characterised in that** the input channel (6, 51, 65) is diminished to reduce its inside diameter so that the average inside diameter of the input channel (6, 51, 65) is smaller than the average inside diameter of the output channel (11, 52, 66).

13. The method according to Claim 12, **characterised in that** the diminution is achieved by reducing the outside diameter of the input channel (6, 51, 65) while maintaining at least approximately the wall thickness, and/or by inserting elevations, bulges or the like into the input channel (6, 51, 65).

14. The method according to one or more of Claims 12 or 13, **characterised in that** the reduction of the inside diameter of the input channel (6, 51, 65) is varied.

15. The method according to one of Claims 12 to 14, **characterised in that** the flow of the fluid is controlled by varying the inside diameter of the input channel (6, 51, 65).

16. The method according to one of Claims 12 to 15, **characterised in that** at least one electrode (8) provided for generating the electrical field in the chamber (7, 54, 62) is cooled.

## Revendications

1. Dispositif (1, 50, 60) se composant au moins d'un canal d'entrée (6, 51, 65) au moins pour introduire un fluide dans une chambre (7, 54, 62) et au moins d'un canal de sortie (11, 52, 66) pour évacuer le fluide de la chambre (7, 54, 62), la chambre (7, 54, 62) présentant au moins deux électrodes (8) pour la génération d'un champ électrique dans la chambre (7, 54, 62), lesquelles sont en contact avec l'espace intérieur (10, 53, 67) de la chambre (7, 54, 62), **caractérisé en ce que** le diamètre intérieur moyen du canal d'entrée (6, 51, 65) est inférieur au diamètre intérieur moyen du canal de sortie (11, 52, 66).

2. Dispositif selon la revendication 1, **caractérisé en ce que** le diamètre intérieur du canal d'entrée (6, 51, 65) est inférieur, au moins à un endroit, au plus petit diamètre intérieur du canal de sortie (11, 52, 66).

3. Dispositif selon la revendication 1 ou 2, **caractérisé en ce que** le canal d'entrée (6, 51, 65) présente au moins un rétrécissement (15, 55, 63) pour la réduction de son diamètre intérieur.

4. Dispositif selon la revendication 3, **caractérisé en ce que** le rétrécissement (15, 55, 63) est réalisé par réduction du diamètre extérieur du canal d'entrée (6, 51, 65) avec une épaisseur de paroi restant sensiblement la même et/ou grâce à des bossages, bourrelets ou similaires disposés à l'intérieur du canal d'entrée (6, 51, 65).

5. Dispositif selon l'une ou plusieurs des revendications 1 à 4, **caractérisé en ce que** le canal de sortie (11, 52, 66) est réalisé en forme d'arc ou de courbe.

6. Dispositif selon l'une ou plusieurs des revendications 1 à 5, **caractérisé en ce que** l'embouchure du canal de sortie (11, 52, 66) est disposée au-dessus de la chambre (7, 54, 62).

7. Dispositif selon l'une ou plusieurs des revendications 1 à 6, **caractérisé en ce que** le canal de sortie (11, 52, 66) et/ou le canal d'entrée (6, 51, 65) est/sont muni(s) d'au moins un clapet, de préférence d'au moins un clapet anti-retour.

8. Dispositif selon l'une ou plusieurs des revendications 1 à 7, **caractérisé en ce que** le canal d'entrée (6, 51, 65) et/ou le canal de sortie (11, 52, 66) est/sont muni(s) d'au moins une électrode de mise à la terre (20, 61, 68).

9. Dispositif selon l'une ou plusieurs des revendications 1 à 8, **caractérisé en ce que** les au moins deux électrodes (8) sont munies d'au moins un dispositif de refroidissement sur leur côté tournant le dos au fluide.

10. Dispositif selon la revendication 9, **caractérisé en ce qu'**une feuille thermo-conductrice, non électroconductrice, est disposée entre l'électrode (8) et le dispositif de refroidissement.

11. Utilisation du dispositif selon l'une ou plusieurs des revendications 1 à 10 pour l'électroporation ou l'électrofusion de cellules, de particules de cellules et/ou de vésicules membranaires selon le procédé par écoulement.

12. Procédé pour la stabilisation du flux d'un fluide à travers une chambre (7, 54, 62), laquelle présente au moins deux électrodes (8) qui sont en contact avec l'espace intérieur (10, 53, 67) de la chambre (7, 54, 62), ainsi qu'au moins un canal d'entrée (6, 51, 65) pour introduire le fluide dans la chambre (7, 54, 62) et au moins un canal de sortie (11, 52, 66) pour évacuer le fluide de la chambre (7, 54, 62), un champ électrique étant généré dans la chambre (7, 54, 62) à l'aide des deux électrodes (8), **caractérisé en ce que** le canal d'entrée (6, 51, 65) est rétréci afin de réduire son diamètre intérieur et que de ce fait, le diamètre intérieur moyen du canal d'entrée (6, 51, 65) est inférieur au diamètre intérieur moyen du canal de sortie (11, 52, 66).

13. Procédé selon la revendication 12, **caractérisé en ce que** le rétrécissement est réalisé par réduction du diamètre extérieur du canal d'entrée (6, 51, 65) avec une épaisseur de paroi restant sensiblement identique et/ou par insertion de bossages, bourrelets ou similaires dans le canal d'entrée (6, 51, 65).

14. Procédé selon l'une ou plusieurs des revendications 12 ou 13, **caractérisé en ce que** l'on fait varier la réduction du diamètre intérieur du canal d'entrée (6, 51, 65).

15. Procédé selon l'une ou plusieurs des revendications 12 à 14, **caractérisé en ce que** le flux du fluide est guidé en faisant varier le diamètre intérieur du canal d'entrée (6, 51, 65).

16. Procédé selon l'une ou plusieurs des revendications 12 à 15, **caractérisé en ce que** l'on refroidit au moins une électrode (8) qui est prévue pour générer le champ électrique dans la chambre (7, 54, 62).
